(19) Europäisches Patentamt / European Patent Office / Office européen des brevets

(11) **EP 2 238 200 B1**

(12) **EUROPEAN PATENT SPECIFICATION**

(45) Date of publication and mention
of the grant of the patent:
**10.09.2014 Bulletin 2014/37**

(51) Int Cl.:
*A23L 2/395* (2006.01)   *A23L 2/52* (2006.01)
*A23F 5/40* (2006.01)   *A23L 1/0522* (2006.01)
*A61K 8/73* (2006.01)   *A61Q 19/10* (2006.01)
*C08L 5/00* (2006.01)   *A23L 2/39* (2006.01)
*A23F 3/30* (2006.01)   *A23F 5/38* (2006.01)
*A23G 1/56* (2006.01)   *A23L 1/054* (2006.01)
*A23L 1/09* (2006.01)   *C08L 3/02* (2006.01)

(21) Application number: **09706638.5**

(22) Date of filing: **12.01.2009**

(86) International application number:
**PCT/EP2009/050248**

(87) International publication number:
**WO 2009/095294 (06.08.2009 Gazette 2009/32)**

(54) **WATER SOLUBLE CARRIER**

WASSERLÖSLICHER TRÄGER

VÉHICULE HYDROSOLUBLE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **01.02.2008 EP 08150980**

(43) Date of publication of application:
**13.10.2010 Bulletin 2010/41**

(73) Proprietors:
• **Unilever PLC
London
EC4Y 0DY (GB)**
Designated Contracting States:
**CY GB IE MT**
• **Unilever N.V.
3013 AL Rotterdam (NL)**

(72) Inventors:
• **INOUE, Chiharu
NL-3133 AT Vlaardingen (NL)**
• **MIAO, Song
Fermoy, Co. Cork (IE)**

(74) Representative: **Askew, Sarah Elizabeth
Unilever Patent Group
Colworth House
Sharnbrook
Bedford MK44 1LQ (GB)**

(56) References cited:
**WO-A-2004/043440    WO-A-2007/009600
WO-A-2008/002851**

**Description**

**TECHNICAL FIELD OF THE INVENTION**

The present invention relates to a water soluble carrier. In particular the present invention relates to an improved water soluble carrier which is suitable for use in blocks such as precursors for tea-based beverages.

**BACKGROUND TO THE INVENTION**

Beverages based on plants such as tea, coffee and cocoa have been popular throughout the world for hundreds of years. Traditionally such beverages are produced by infusing beverage plant material (e.g. tea leaves, coffee beans etc.) in hot water and separating the aqueous plant extract from the remaining insoluble plant material. It has also been traditional to vary the flavour of the beverages by the inclusion of aromatic plant material such as mint leaves, lemon slices and vanilla pods.

Today such beverages can be prepared by more convenient methods which dispense with the need for manipulation of insoluble plant material by the end-user or consumer. In particular, the beverages can be prepared from instant powders or granules which are free from insoluble plant extract and so dissolve rapidly and completely on contact with hot water.

A more recent development has been the introduction of beverage precursor blocks.

JP 61-152238 A (Amano Jitsugyo KK) discloses the production of dried tea in pellet form wherein tea is extracted with hot water, concentrated, blended with a saccharide (preferably dextrin, etc., having low sweetness, etc.), and injected into a mold having a large number of pellet-like dents. The extracted solution is frozen in an injected state in the mold, taken out from the mold, and lyophilized while being in a frozen state.

WO 2007/009600 (Unilever PLC et al.) discloses a beverage precursor in the form of a block weighing at least 0.2 g, and comprising plant extract (such as tea, coffee or cocoa solids) and one or more insoluble inclusions (such as a fruit or leaf). In a preferred embodiment, the block comprises water-soluble carrier which enhances the fracture strength and/or flavour delivery of the blocks. The preferred biopolymer for use in the water soluble carrier of WO 2007/009600 is maltodextrin (i.e. hydrolysed starch) owing to its high water solubility, clean taste and fragrance-binding properties. The DE (dextrose equivalent) of the maltodextrin is preferably between 4 and 60, more preferably between 10 and 40.

Whilst maltodextrins are suitable components for use in a water soluble carrier, we have found that blocks made from such carriers can still exhibit excessive friability. To this end we have sought a water soluble carrier which retains many of the benefits of maltodextrin such as rapid dissolution in water and good flavour binding, but which is capable of imparting improved friability to blocks manufactured therefrom.

Pullulan is a natural water-soluble polysaccharide, produced from starch by fermentation. Pullulan consists of maltotriose units linked through alpha 1,6-glucosidic bonds.

The use of pullulan in water soluble carriers is known. For example, WO 2004/043440 discloses solid dosage forms of active agents and pullulan wherein the solid dosage form has a friability of less than about 1%.

Mixtures of pullulan and saccharides are also known. For example, US 5,411,945 (HAYASHIBARA BIOCHEM LAB) discloses a binder which comprises as a main ingredient pullulan and saccharide(s) wherein the weight ratio of said pullulan to saccharide(s) is in the range of 85:15 to 65:35, based on the weight of the dry solid.

We have now found that the combination of a specific type of maltodextrin with a selective amount of pullulan results in a water soluble carrier which has exceptional properties, and is especially suited for use in blocks such as beverage precursors for tea based beverages.

**DEFINITIONS**

Dextrose Equivalent

The dextrose equivalent (DE) of a maltodextrin is the total reducing power, expressed as dextrose (D-glucose) and calculated on a dry weight basis. It is a well-known term used in the field.

The DE value effectively represents the amount of glucose units present as reducing end groups. Therefore, as each molecule has only a single end group, DE indicates the degree of polymerisation (DP) of a dextrin molecule, i.e. the number of monosaccharide units in the molecule. In fact the DE can be related to DP by the following equation (1):

$$DE = 100 \ / \ DP \qquad\qquad (1)$$

Thus, the DE of pure dextrose is 100; the DE of pure maltose is 50 and the DE of a dextrin polymer having a DP of 100 is 1. Because maltodextrin consists of a mixture of glucose polymers having different lengths, the DE is an average value and can be related to the number average molecular weight ($M_n$) by the following equation (2) (Chirife J, Buera MP, "A simple model for predicting the viscosity of sugar and oligosaccharide solutions", Journal of Food Engineering, 1997, 33, pp. 221-226):

$$M_n \ (\mathrm{g\ mol^{-1}}) = 18016 \ / \ DE \qquad\qquad (2)$$

Therefore, the higher the DE, the higher the level of monosaccharides and short chain polymers in a maltodextrin.

Tea

"Tea" for the purposes of the present invention means material from *Camellia sinensis* var. *sinensis* and/or *Camellia sinensis* var. *assamica.*

Beverage

As used herein the term "beverage" refers to a substantially aqueous drinkable composition suitable for human consumption.

Plant extract

As used herein, the term "plant extract" refers to solids extractable in boiling water from beverage plant material such as tea leaves, coffee beans or cocoa beans. Thus a plant extract contains most of the active and flavour components of a beverage. Typically the plant extract will comprise components such as caffeine, polyphenols (e.g. flavonols [catechins], flavonol glycosides, leucoanthocyanins, phenolic acids) and aroma compounds (e.g. acids, alcohols, aldehydes, esters, hydrocarbons, lactones). Such plant extracts are well known in the art and are usually obtained by drying an aqueous extract obtained from treating plant material with hot (typically at least 30°C, preferably at least 60°C, more preferably at least 90°C) water. The plant material may be raw or may have undergone a processing step such as roasting, withering and/or fermentation prior to extraction.

For example the plant extract may be tea solids extracted from green leaf tea, black leaf tea, oolong leaf tea or mixtures thereof. The method of preparing such leaf teas is well known to those skilled in the art. Generally, to prepare black leaf tea, fresh green leaves of the plant *Camellia sinensis* are withered (subjected to mild drying), comminuted, fermented (in which enzymes in the leaf tea oxidise various substrates to produce brown-coloured products) and then fired (to dry the tea leaves). Green leaf tea is not exposed to the fermentation process. Partial fermentation may be used to produce intermediate-type teas known as "oolong" tea. Plant extract may be prepared from any of these leaf materials, e.g. by contacting the leaf material with hot water, separating out the insoluble material and drying the remaining extract.

Beverage Precursor

A beverage precursor is defined as a fabricated composition suitable for preparing a beverage.

Block

As used herein, the term "block" is defined as a cohesive solid mass and thus encompasses (but is not limited to) such terms as cube, tablet, pellet, ingot, bar and brick.

Distributing

References herein to "distributing" a substance in an aqueous medium are to be understood as meaning that the substance is mixed with the aqueous medium by dissolving and/or dispersing the substance in the aqueous medium.

Water-soluble carrier

The term "water-soluble carrier" is defined as a solid which is substantially soluble in boiling water.

Fracture strength

The fracture strength of a block is defined as the stress at fracture measured as follows:

At least three representative cuboid samples measuring 3.25 x 2.3 x 2.1 cm of the block are tested at 20°C using an Instron™ Universal Testing machine. A flat plate compression test is performed on each sample in the direction perpendicular to the 3.25 x 2.3 cm faces of the sample. A crosshead speed of 100 mm/min is used to a displacement of 15 mm, with a data sampling rate of 50 points/s. The maximum force recorded is then taken as the Failure Force (i.e. maximum force of the failure peak). The Fracture Strength of the block is calculated by dividing the mean Failure Force of the samples by the cross-sectional Area (3.25 x 2.3 cm) i.e. Fracture Strength (kN m$^{-2}$) = Failure Force (kN) / Area (m$^2$) = Failure Force (kN) / 0.00074 m$^2$.

Friability

The friability of a block is defined as the weight percentage of fines resulting from controlled pulverization of a block and is determined as follows:

Five representative cylindrical samples with a length of 2.8 cm and a diameter of 2.6 cm are pulverized together in a rotating cylinder. The rotating cylinder has an internal length of 10 cm and an internal diameter of 10 cm. The five samples are weighed and then placed, along with 20 ceramic balls (each of diameter 10 mm), in the cylinder. The cylinder is then rotated about its longitudinal axis at 100 rpm for 2 minutes with the longitudinal axis of the cylinder kept at 30° to horizontal throughout. The contents of the cylinder are then sieved through a 3.5 (Tyler) mesh sieve (5.6 mm aperture size) with material passing through the sieve being referred to as "fines". The Friability of the block is calculated as the mass of fines as a proportion of the total mass of the five samples before pulverization i.e. Friabilty (wt%) = 100 × Mass of Fines / Total mass of five blocks. The Friability is quoted as the mean value of three independent tests, each test performed on 5 representative samples.

Freezing Rate

As defined herein, the freezing rate of a portion of premix is defined as follows:

$$\text{Freezing rate } = 10 \text{ K } / \ t \text{ min,} \qquad\qquad (3)$$

wherein t is the time taken for the centre of the portion to cool from a temperature of 0°C to -10°C.

Insoluble inclusions

As used herein, the term "insoluble inclusion" refers to a coherent mass which is substantially insoluble in boiling water. The insoluble inclusion should be of a size and solubility such that the insoluble inclusion is visible, as a discrete piece or pieces, in a beverage prepared by contacting a block comprising the inclusion with boiling water, after substantially all of the water-soluble carrier and plant extract (if present) included in the block have dissolved and/or dispersed in the boiling water.

Size

Unless stated otherwise, the size of a block or inclusion is quoted as the maximum linear dimension of the block or inclusion, i.e. the maximum length in any dimension.

SUMMARY OF THE INVENTION

We have found that the use of maltodextrin with a DE of from 4 to 40 results in a water soluble carrier which dissolves rapidly in boiling water, has excellent flavour binding properties and has low sweetness. In addition, we have found that including pullulan in the carrier increases the mechanical strength and decreases the friability of blocks containing the carrier. However, we have also found that limiting the amount of pullulan such that the weight ratio of maltodextrin to pullulan is greater than 3 is necessary in order to retain the ability of the carrier to dissolve rapidly.

Thus, in a first aspect, the present invention provides a water-soluble carrier comprising:

a) maltodextrin having a dextrose equivalent (DE) in the range of 4 to 40; and
b) pullulan;

wherein the weight ratio of maltodextrin to pullulan is greater than 3.

The carrier is especially suited for use in blocks such as beverage precursors for tea based beverages.

## DETAILED DESCRIPTION

Water-Soluble Carrier

The water-soluble carrier of the invention comprises pullulan and a specific maltodextrin in a specific weight ratio.

*Maltodextrin*

Maltodextrin (also known as hydrolysed starch) has high water solubility, clean taste and fragrance-binding properties. The DE of the maltodextrin is preferably not too high as to impart a sweet taste and hygroscopic character to blocks made therefrom, nor too low such that the maltodextrin is insoluble. Furthermore, maltodextrins with too high DE introduce unwanted friability to blocks made therefrom. Thus we have found it necessary to use maltodextrin with a DE of between 4 and 40, more preferably between 10 and 30 and most preferably from 15 to 25.

*Pullulan*

Pullulan imparts mechanical strength and decreases the friability of blocks containing the carrier. However, we have also found that limiting the amount of pullulan such that the weight ratio of maltodextrin to pullulan is greater than 3 is necessary in order to retain the ability of the carrier to dissolve rapidly. Even better rates of dissolution can be attained if the ratio of maltodextrin to pullulan is greater than 5, more preferably greater than 8. Too low a level of pullulan can, however, result in excessive friability of blocks made from the carrier. Thus it is preferred that the weight ratio of maltodextrin to pullulan is less than 30, more preferably less than 20 and most preferably less than 15.

In general, higher molecular weight polymers are more effective at imparting mechanical strength to blocks made from the carrier, thus it is preferred that the pullulan has a weight-average molecular weight ($M_w$) of at least $1 \times 10^5$ g mol$^{-1}$. Too high a molecular weight may impair the water solubility of the carrier, however. Thus it is also preferred that the pullulan has a weight-average molecular weight of less than $1 \times 10^7$ g mol$^{-1}$, more preferably less than $1 \times 10^6$ g mol$^{-1}$.

*Other Components*

The water-soluble carrier may comprise other components in addition to maltodextrin and pullulan. It is preferred, however, that the major part of the carrier is made up of the maltodextrin and pullulan. In particular, it is preferred that the carrier comprises the pullulan and maltodextrin in a total amount of at least 50% by dry weight of the carrier, more preferably at least 80% by dry weight of the carrier, and most preferably from 90% to 100% by dry weight of the carrier.

The carrier may comprise further polymers, especially biopolymers, such as non-starch polysaccharides and/or proteins. It is preferable, however, that the carrier is substantially free from gelatin as gelatin provides a "gummy" mouthfeel to foodstuffs and is unsuitable for many consumers such as vegetarians.

It is preferable that the carrier is inert with respect to taste and flavour. In particular, large amounts of salt should be avoided, i.e. the carrier should comprise less than 1% salt, more preferably less than 0.5 and most preferably less than 0.1% salt by dry weight of the carrier. This is because not only does salt interfere with taste but it may also decrease the solubility of other components of the carrier and/or block. The carrier may, however, comprise sugars (i.e. mono- and/or di-saccharides) in an amount of up to 50% by weight of the carrier.

The carrier may comprise non-fat milk solids in an amount of at least 1% by weight of the carrier, or even in an amount of between 10 and 50% by weight of the carrier. The milk solids may be derived from any suitable source such as milk, skimmed milk and/or whey.

Block Containing the Carrier

The carrier is especially suited for use in blocks. The carrier may be used in blocks for a range of applications where water-solubility is desirable. For example the block could be a pharmaceutical preparation, a cleansing composition or a precursor for a foodstuff, especially a beverage precursor.

*Composition of the Block*

In order to provide optimum water-solubility and mechanical strength to the block, it is preferable that a large proportion of the block comprises the water-soluble carrier, i.e. the block comprises at least 40% water-soluble carrier by weight of the block, more preferably at least 50% and optimally from 60 to 90%.

In order to ensure maximum storage stability, it is preferred that the block is dried. In particular, it is preferred that the water content of the block is less than 30% by weight of the block, more preferably less than 15% and most preferably in the range 1 to 10%.

Where the block is a beverage precursor block, it is particularly preferred that the block comprises plant extract. For optimum convenience, the beverage precursor block should contain sufficient plant extract such that no more than 5 blocks are required to prepare a single 200 g serving of beverage. Preferably the amount of plant extract in the block is such that only 1 or 2 blocks are required to prepare a single serving. Most preferably a single block is sufficient to prepare a single serving. Thus it is preferred that the block comprises plant extract in an amount of at least 2% by weight of the block, more preferably at least 5% and optimally from 10 to 50%. Preferably the block comprises at least 0.05 g of plant extract, more preferably at least 0.1 g and optimally between 0.2 and 2 g.

Examples of plant extract include tea solids, coffee solids, cocoa solids, camomile solids, nettle solids, mint solids, rooibos solids, fruit solids and mixtures thereof. Particularly preferred plant extracts are fruit solids, tea solids, coffee solids, cocoa solids and mixtures thereof. The beverage precursor block most preferably comprises tea solids, and in one particularly preferred embodiment the only plant extract present is tea solids.

The beverage precursor blocks of the present invention are suitable for delivering insoluble inclusions. Thus, in a preferred embodiment the block comprises one or more insoluble inclusions.

As the insoluble inclusions should be of such a size that they are visible in a beverage, it is preferred that at least one of the insoluble inclusions has a size of at least 1 mm, more preferably 5 mm and most preferably at least 10 mm. In order that the inclusions are substantially supported by the strength of the water-soluble carrier in the block, it is preferred that all of the inclusions are of a size substantially the same or less than that of the remainder of the block. In particular it is preferred that the inclusions are smaller than 100 mm, more preferably smaller than 50 mm and most preferably smaller than 30 mm.

It is also preferable that the block comprises the inclusions in an amount of less than 60% by weight of the block, more preferably less than 50% and most preferably in an amount between 5 and 40%.

The block may comprise a plurality of inclusions, e.g., at least 2 inclusions, more preferably from 3 to 10 inclusions. In a preferred embodiment, however, the block comprises a single inclusion.

The inclusion may be any suitable insoluble entity that adds to the interest, excitement and/or enjoyment of the finished beverage and may be, for example, a confectionery piece such as a gelled sweet. In a particularly preferred embodiment the insoluble inclusions comprise plant material. The plant material may be selected from the group consisting of whole fruit, fruit pieces, whole leaves, leaf pieces, whole pods, pod pieces, whole seeds, seed pieces, whole beans, bean pieces, whole roots, root pieces, whole nuts, nut pieces, whole flowers, flower pieces and mixtures thereof. Particularly preferred are fruits (such as whole berries and/or citrus slices), pods (such as whole and/or parts of vanilla pods) and/or leaves (such as whole and/or parts of tea and/or mint leaves).

The inclusions may comprise plant material derived from the same plant as the plant extract and may, for example, comprise whole and/or parts of tea leaves (especially green tea leaves). In a preferred embodiment, however, the inclusions comprise at least 50% (more preferably at least 75%) by weight of the inclusions of a plant material derived from a plant other than the plant or plants from which the plant extract is derived. Most preferred is that the inclusions are substantially free from plant material derived from the same plant as the plant extract. In particular the inclusions may comprise less than 10% by weight of the inclusions (more preferably less than 1%) of tea leaves, coffee beans, cocoa beans or parts thereof.

The beverage precursor block may also comprise other minor additives and actives, typically in an amount of less than 10% by weight of the block, and preferably in an amount of between 0.1 and 5% by weight of the block. Examples of minor additives include flavour compositions, spices and colourings. Examples of actives include nutritional actives such as minerals, vitamins and bioactive peptides/amino acids (such as theanine). The beverage precursor block of this invention is particularly suited for use as a delivery vehicle for nutritional and/or health actives.

*Properties of the Block*

It is preferred that the block has a relatively large mass to provide increased convenience compared to powdered or granular substances. It is particularly preferred that the block has a dry mass of at least 0.2 g, more preferably at least 1 g, most preferably at least 2 g. In order to avoid the blocks becoming too large for convenient storage (especially where the blocks have a low density) it is preferred that the block has a dry mass of less than 300 g, more preferably less than 100 g and most preferably less than 25 g.

The block may be any suitable shape but in a preferred embodiment, the block has a moulded shape of, for example, a fruit, leaf, nut, bean, heart, letter, number or logo. Alternatively, the block may comprise a plurality of sub-blocks, with neighbouring sub-blocks being joined by an area of relative weakness. Such an arrangement allows for the user to easily detach an intact sub-block from the remainder of the sub-blocks.

The detached sub-block may then be used (for example, to prepare a beverage) while the remaining sub-blocks stay as a single block for easy storage.

It is preferred that the water-soluble carrier and other components of the block should dissolve rapidly such that the block is dissolved in boiling water in less than 30 s without mechanical agitation (e.g. stirring). More preferably the block dissolves in less than 10 s and most preferably less than 5 s. Optimally the block dissolves instantaneously in an excess of boiling water (i.e., in less than 2 s).

In order to provide especially rapid dissolution of the block in an aqueous medium it is preferable that the block has a low density and/or a high porosity. Preferably the density of the block is less than 0.5 g ml$^{-1}$, more preferably less than 0.4 g ml$^{-1}$ and optimally between 0.05 and 0.3 g ml$^{-1}$.

To minimise friability of the blocks, it is preferable that the block has a fracture strength of at least 50 kPa, more preferably at least 70 kPa, and most preferably in the range 80 to 1000 kPa. Alternatively or additionally, the block may have a friability of less than 30% by weight, more preferably from 1 to 20%.

*Process for Manufacturing the Block*

The block of the present invention may be manufactured by any suitable process including tabletting, agglomeration and combinations thereof. However in a preferred embodiment, the block is formed without finely dividing the premix into grains or granules and so the resulting block has a more coherent continuous solid structure which gives superior mechanical strength. Thus the block is preferably manufactured by a process comprising the steps of:

(i) providing an aqueous premix comprising the water-soluble carrier;
(ii) drying the premix; and
(iii) dividing out a cohesive portion of the premix, the portion having a dry mass of at least 0.2 g and the portion forming the block when dried.

The aqueous premix may be provided in step (i) by any suitable means and may, for example, be formed by distributing (and more preferably dissolving) the pullulan and maltodextrin in water.

Where the block comprises plant extract, the premix may be formed by adding the water-soluble carrier to aqueous plant extract directly obtained from treating plant material with an aqueous medium. By "directly obtained" is meant that the aqueous extract is obtained without drying to a solid, but does not exclude the possibility of the aqueous extract having undergone a concentration step, e.g. by evaporation under vacuum, reverse osmosis or other suitable method. Preferably, however, step (i) comprises distributing the plant extract and water-soluble carrier in an aqueous medium thereby to form the aqueous premix as this provides for a more flexible process and allows for better control of the composition of the raw materials and therefore of the premix.

In order to provide especially rapid dissolution of the final block in an aqueous medium it is preferable that the block has a low density and/or a high porosity. Such a low density and high porosity is provided to some extent by voids formed by loss of water during the drying step (ii). Thus it is preferable that the aqueous premix comprises at least 50% water by weight of the premix, more preferably at least 60% and most preferably between 70 and 95%. Also, because formation of the block does not require a compression step (cf. a tabletting process) then these voids remain in the final block. Thus the present process dispenses with the need for dispersing aids such as effervescing salts and preferably the block is substantially free of dispersing aids, more preferably the block contains less than 1% dispersing aid by weight of the block, most preferably less than 0.1%.

In order to decrease the density of the block still further, the aqueous premix may be foamed, e.g. by whipping with air or use of a soluble gas (such as $CO_2$ or $N_2O$), to an overrun of at least 50%, preferably between 100 and 300%. However, we have found that satisfactory densities can be obtained without the need for foaming of the aqueous premix.

In order to ensure maximum storage stability, it is preferred that the premix is dried in step (ii) such that the water content of the block is less than 30% by weight of the block, more preferably less than 15% and most preferably in the range of 1 to 10%.

Step (ii) may be conveniently accomplished by freeze-drying of the premix. This is because sublimation of water during freeze-drying results in a particularly low density, highly porous block. We have found that when freeze-drying is used in the process of the present invention, it is advantageous to employ especially high rates of freezing, otherwise the mechanical strength of the blocks may become too low such that the blocks are extremely friable. This effect is particularly apparent when blocks comprising high levels of plant extract are prepared. Thus it is preferred that the premix is frozen at a rate of at least 0.15 K min$^{-1}$, more preferably in the range of from 0.2 to 1 K min$^{-1}$ and optimally

in the range of from 0.25 to 0.4 K min$^{-1}$.

The interaction between the carrier and the plant extract also appears to occur, to some extent, even prior to freezing. Therefore it is preferable that the time between steps (i) and (ii) is kept to minimum, i.e., drying should commence no more than 5 hours after forming the aqueous premix, more preferably no more than 2 hours, even more preferably no more than 1 hour and optimally between 0.1 and 30 minutes.

The cohesive portion of premix may be divided out by any suitable means provided that the portion itself is not finely divided such that its dry mass is below 0.2 g. It is preferred that the portion of premix used to form the block has a dry mass of at least 1 g, more preferably at least 2 g. It is also preferred that the portion has a dry mass of less than 300 g, more preferably less than 100 g and most preferably less than 25 g.

In one embodiment, the steps of the process may be performed in the order (i), then (ii) and then (iii), e.g., the premix may be dried in bulk and the portion cut, as the block, from the dried premix. The process steps may also be performed in the order (i), then (iii) and then (ii), e.g. the portion may be divided out by dosing the portion as liquid premix into a mould prior to drying. Alternatively, the premix may be frozen in bulk (e.g. by slush-freezing) and the portion cut from the frozen premix prior to drying in step (ii) (e.g. by freeze-drying). An advantage of dividing out the portion prior to drying is that the liquid (or slush-frozen) portion may be easily moulded into any desired shape with little wastage of premix.

*Packaging*

The block is preferably packaged, more preferably along with one or more additional blocks. In a particularly preferred embodiment the block is packaged with one or more substantially identical blocks such that the package comprises a plurality of substantially identical blocks. By "substantially identical" is meant that the size, shape and composition of the blocks are substantially invariant between the blocks. Most preferable is that the package comprises a plurality of blocks with a substantially monodisperse size distribution, i.e. at least 50% of the blocks are the same size, more preferably at least 70% and optimally all of the blocks are the same size.

Where the block is a beverage precursor, the package may comprise the beverage precursor blocks in an amount of a single serving.

An advantage of the beverage precursor blocks of the present invention is that the blocks are not required to comprise insoluble beverage material that requires separation from the plant extract by the consumer or end user. As such it is not necessary to include the beverage block in an infusion package such as a tea bag. Thus in a preferred embodiment, the package comprises the beverage precursor block which is not enclosed in an infusion package.

*Use of the Block*

The carrier may be used in blocks for a range of applications where water-solubility is desirable.

Where the block is a pharmaceutical preparation it will typically comprise a pharmaceutically active compound in addition to the carrier. The pharmaceutically active compound is typically administered to an individual by the individual orally ingesting the block.

Where the block is a cleansing preparation it may be, for example, a laundry tablet, dishwashing tablet, or skin cleansing composition. Cleansing blocks typically comprise a detergent in addition to the carrier. In use, the block is typically dissolved in water and the resulting aqueous solution used to remove dirt from a substrate such as, for example, textile, cookware or skin.

In a most preferred embodiment the block is a beverage precursor, especially for tea based beverages.

The beverage precursor blocks of the present invention are intended for use in preparing a beverage. The beverage is typically prepared by a method comprising the steps of:

(A) providing the beverage precursor block; and
(B) contacting the block or a part thereof with an aqueous medium.

Typically the beverage comprises at least 85% water, more preferably at least 90% and optimally between 95 and 98% by weight of the beverage. Preferably the beverage is a coffee-based beverage, a tea-based beverage and/or a cocoa-based beverage. Most preferably the beverage is tea.

The aqueous medium may be any edible liquid but is preferably selected from water and/or milk. Preferably also, during step (B) the aqueous medium is hot and has a temperature of at least 30°C, more preferably at least 60°C and most preferably between 90 and 100°C. In an alternative embodiment, the aqueous medium is cold and has a temperature of less than 30°C, more preferably less than 15°C and most preferably between 0 and 10°C.

In a preferred embodiment, the block and aqueous medium are contacted in step (B) in a manner such that the plant extract and water-soluble carrier are distributed throughout the aqueous medium and the insoluble inclusions (if present) are visible as a discrete piece or pieces in the beverage.

In a preferred embodiment the beverage is a single serving and at least 100 g (preferably between 150 and 250 g) of the aqueous medium is contacted with less than 5 of the beverage blocks, more preferably less than 3 and most preferably a single block or part thereof.

The beverage precursor block (or part thereof) may be crumbled and/or disintegrated prior to step (B) to increase the surface area of beverage precursor in contact with the aqueous medium in step (B) and thus increase the rate of distribution therein.

If the beverage precursor block comprises a plurality of sub-blocks, then step (B) may comprise separating at least one of the sub-blocks from the beverage precursor block and contacting the at least one separated sub-block with the aqueous medium.

## EXAMPLES

The present invention will be further described with reference to the following examples.

### Example 1

This Example demonstrates the influence of the composition of the water soluble carrier on the friability and solubility of tea-based beverage precursor blocks.

*Materials*

Maltodextrin was GLUCIDEX™21 supplied by Roquette UK Ltd (Corby, UK) and had a DE of 20.

Pullulan was from Hayashibara (Epsom, UK) and had a weight-average molecular weight in the range $1 \times 10^5$ to $1 \times 10^6$ g mol$^{-1}$.

Instant tea powder was PG Tips™ Instant supplied by Unilever Bestfoods UK Ltd.

*Formulations*

Blocks were prepared from six aqueous premixes. The premix formulations are given Table 1. In all cases the balance of the formulation was water. Also shown in Table 1 is the weight ratio (R) of maltodextrin to pullulan for each formulation.

TABLE 1

| Ingredient (%w/w) | Formulation | | | | | |
|---|---|---|---|---|---|---|
| | **1*** | **2*** | **3** | **4** | **5** | **6*** |
| Maltodextrin | 10 | 15 | 18 | 18.5 | 19 | 20 |
| Pullulan | 10 | 5 | 2 | 1.5 | 1 | 0 |
| Tea Powder | 3.5 | 3.5 | 3.5 | 3.5 | 3.5 | 3.5 |
| *R* | 1 | 3 | 9 | 12.3 | 19 | -- |
| *comparative | | | | | | |

*Process*

Aqueous premix was prepared by first making a dry blend of the maltodextrin, tea powder and pullulan (if needed). The required amount of cold distilled water was then placed in a beaker situated in a water bath on a stirrer-hotplate. The stirrer was activated and the dry blend slowly added to the agitated water. After 5 minutes of continuous stirring the heater was activated and the water bath heated to 85°C. The contents of the beaker were continuously stirred at this temperature until all of the blend had dissolved (usually 10-30 minutes at 85°C). The beaker was then removed from the hot plate and the aqueous premix rapidly cooled to 20°C by running tap water around the outside of the beaker.

The aqueous premix was then poured into cylindrical moulds, each with an internal length of around 28 mm and internal diameter of around 26 mm. The moulds were then placed in a conventional air-blast freezer operating at a temperature of -35°C and blast-frozen for 2 hours. The frozen blocks were then removed from the moulds and placed in the chamber of a freeze-drier. Air was removed from the freeze-drier until the vacuum reached 0.2 mbar, at which point the temperature of the chamber was increased to +20°C. The total drying time was around 40 hours.

The dried blocks were stored at room temperature (~20°C) in a sealed container prior to testing.

*Tests*

Blocks made from each formulation were tested for friability and rate of dissolution.

For testing the rate of dissolution, an intact block was first placed in a beaker, and then 200 ml of freshly boiled water (about 90-100°C) was poured onto the block. No agitation was used. Blocks were considered to be instantly soluble if no undissolved matter was visible in the beverage after 10 s.

*Results*

The results of the tests are set forth in Table 2.

TABLE 2

| Formulation | $R$ | Friability (%) | Instant Solubility |
|---|---|---|---|
| 1* | 1 | 4 | NO |
| 2* | 3 | 8 | NO |
| 3 | 9 | 17 | YES |
| 4 | 12.3 | 18 | YES |
| 5 | 19 | 20 | YES |
| 6* | -- | 50 | YES |
| * comparative | | | |

The data in Table 2 illustrate that excellent friability (i.e. 20% or less) is obtained with blocks wherein the water-soluble carrier comprises pullulan (formulations 1-5). Blocks wherein the water-soluble carrier comprises high levels of pullulan (formulations 1 and 2), however, do not show instant solubility.

**Claims**

1. A water-soluble carrier comprising:

    a) maltodextrin having a dextrose equivalent (DE) in the range of 4 to 40; and
    b) pullulan;

    wherein the weight ratio of maltodextrin to pullulan is greater than 3 and is less than 30.

2. A water-soluble carrier according to claim 1 wherein the DE of the maltodextrin is in the range of 10 to 30.

3. A water-soluble carrier according to claim 1 or claim 2 wherein the carrier comprises the pullulan and maltodextrin in a total amount of at least 50% by dry weight of the carrier, preferably at least 90% by dry weight of the carrier.

4. A block comprising the water soluble carrier of any one of the preceding claims.

5. A block according to claim 4 wherein the block has a dry mass of at least 0.2 g, preferably from 1 to 300 g.

6. A block according to claims 4 or claim 5 wherein the water-soluble carrier is present in an amount of at least 40% by weight of the block.

7. A block according to any one of claims 4 to 6 wherein the block has a density in the range of 0.05 to 0.5 g ml$^{-1}$.

8. A block according to any one of claims 4 to 7 which is a beverage precursor block and which additionally comprises plant extract.

9. A beverage precursor block according to claim 8 wherein the plant extract is selected from the group consisting of tea solids, coffee solids, cocoa solids, fruit solids and mixtures thereof.

10. A beverage precursor block according to claim 9 wherein the plant extract comprises tea solids.

11. A beverage precursor block according to any one of claims 8 to 10 wherein the plant extract is present in an amount of at least 2% by weight of the block.

12. A block according to any one of claims 4 to 11 which is obtainable by a process comprising the steps of:

(i) providing an aqueous premix comprising the water-soluble carrier;
(ii) drying the premix; and
(iii) dividing out a cohesive portion of the premix, the portion having a dry mass of at least 0.2 g and the portion forming the block when dried.

13. A block according to claim 12 wherein step (ii) comprises freeze-drying of the premix.


**Patentansprüche**

1. Wasserlöslicher Träger,
der Folgendes aufweist:

a) Maltodextrin mit einem Dextrose-Äquivalent (DE) im Bereich von 4 bis 40; und
b) Pullulan;

wobei das Gewichtsverhältnis zwischen Maltodextrin und Pullulan mehr als 3 und weniger als 30 beträgt.

2. Wasserlöslicher Träger nach Anspruch 1,
wobei das DE des Maltodextrins im Bereich von 10 bis 30 liegt.

3. Wasserlöslicher Träger nach Anspruch 1 oder 2,
wobei der Träger Pullulan und Maltodextrin in einer Gesamtmenge von mindestens 50 %, vorzugsweise mindestens 90 % aufweist, und zwar auf das Trockengewicht des Trägers bezogen.

4. Block,
der den wasserlöslichen Träger nach einem der vorstehenden Ansprüche aufweist.

5. Block nach Anspruch 4,
wobei der Block eine Trockenmasse von mindestens 0,2 g, vorzugsweise von 1 bis 300 g aufweist.

6. Block nach Anspruch 4 oder 5,
wobei der wasserlösliche Träger in einer Menge von mindestens 40 Gew.-% des Blocks vorliegt.

7. Block nach einem der Ansprüche 4 bis 6,
wobei der Block eine Dichte im Bereich von 0,05 bis 0,5 g·ml$^{-1}$ aufweist.

8. Block nach einem der Ansprüche 4 bis 7,
der ein Block in Form einer Vorstufe für ein Getränk ist und der ferner Pflanzenextrakt aufweist.

9. Block in Form einer Vorstufe für ein Getränk nach Anspruch 8,
wobei der Pflanzenextrakt aus der Gruppe ausgewählt ist, die aus Teefeststoffen, Kaffeefeststoffen, Kakaofeststoffen, Fruchtfeststoffen und Gemischen davon besteht.

10. Block in Form einer Vorstufe für ein Getränk nach Anspruch 9,
wobei der Pflanzenextrakt Teefeststoffe aufweist.

11. Block in Form einer Vorstufe für ein Getränk nach einem der Ansprüche 8 bis 10, wobei der Pflanzenextrakt in einer Menge von mindestens 2 Gew.-% des Blocks vorliegt.

12. Block nach einem der Ansprüche 4 bis 11,
der nach einem Verfahren erhalten werden kann, das die folgenden Schritte aufweist:

(i) Bereitstellen eines wässrigen Vorgemischs, das den wasserlöslichen Träger aufweist;
(ii) Trocknen des Vorgemischs; und
(iii) Heraustrennen einer kohäsiven Portion des Vorgemischs, wobei die Portion eine Trockenmasse von mindestens 0,2 g hat und die Portion den Block bildet, wenn sie getrocknet ist.

13. Block nach Anspruch 12,
wobei der Schritt (ii) das Gefriertrocknen des Vorgemischs aufweist.

**Revendications**

1. Support soluble dans l'eau comprenant :

a) de la maltodextrine présentant un équivalent dextrose (DE) dans l'intervalle de 4 à 40 ; et
b) du pullulane ;

dans lequel le rapport massique de maltodextrine au pullulane est supérieur à 3 et inférieur à 30.

2. Support soluble dans l'eau selon la revendication 1 dans lequel le DE de la maltodextrine se trouve dans l'intervalle de 10 à 30.

3. Support soluble dans l'eau selon la revendication 1 ou la revendication 2, dans lequel le support comprend le pullulane et la maltodextrine dans une quantité totale d'au moins 50 % en masse sèche du support, de préférence d'au moins 90 % en masse sèche du support.

4. Bloc comprenant le support soluble dans l'eau selon l'une quelconque des revendications précédentes.

5. Bloc selon la revendication 4, dans lequel le bloc présente une masse sèche d'au moins 0,2 g, de préférence de 1 à 300 g.

6. Bloc selon la revendication 4 ou la revendication 5, dans lequel le support soluble dans l'eau est présent dans une quantité d'au moins 40 % en masse du bloc.

7. Bloc selon l'une quelconque des revendications 4 à 6, dans lequel le bloc présente une densité dans l'intervalle de 0,05 à 0,5 g ml$^{-1}$.

8. Bloc selon l'une quelconque des revendications 4 à 7, lequel est un bloc de précurseur de boisson et lequel comprend de plus un extrait de plante.

9. Bloc de précurseur de boisson selon la revendication 8, dans lequel l'extrait de plante est choisi dans le groupe constitué de matières solides de thé, de matières solides de café, de matières solides de cacao, de matières solides de fruits et de mélanges de celles-ci.

10. Bloc de précurseur de boisson selon la revendication 9, dans lequel l'extrait de plante comprend des matières solides de thé.

11. Bloc de précurseur de boisson selon l'une quelconque des revendications 8 à 10, dans lequel l'extrait de plante est présent dans une quantité d'au moins 2 % en masse du bloc.

12. Bloc selon l'une quelconque des revendications 4 à 11, qui peut être obtenu par un procédé comprenant les étapes consistant :

(i) à fournir un prémélange aqueux comprenant le support soluble dans l'eau ;

(ii) à sécher le prémélange ; et
(iii) à diviser une partie cohésive du prémélange, la portion présentant une masse sèche d'au moins 0,2 g et la portion formant le bloc lorsqu'elle est séchée.

**13.** Bloc selon la revendication 12, dans lequel l'étape (ii) comprend la lyophilisation du prémélange.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 61152238 A
- WO 2007009600 A, Unilever PLC
- WO 2004043440 A
- US 5411945 A

**Non-patent literature cited in the description**

- **CHIRIFE J ; BUERA MP.** A simple model for predicting the viscosity of sugar and oligosaccharide solutions. *Journal of Food Engineering,* 1997, vol. 33, 221-226, Journal of Food Engineering